# EUROPEAN PATENT APPLICATION

(11) **EP 1 269 969 A1**
(43) Date of publication of application: **02.01.2003**
(21) Application number: 01917658.5
(22) Date of filing: 30.03.2001
(51) Int. Cl.: A61K 7/00

(54) **COSMETIC COMPOSITIONS**

(30) Priority: 03.04.2000 JP 2000100322
(71) Applicant: Ajinomoto Co., Inc., Tokyo 104-0031 (JP)
(72) Inventor: MIKAMI, Naoko, AMINO SCIENCE LABORATORIES, Kawasaki-shi, Kanagawa 210-8681 (JP); YOKOTA, Hirofumi, AMINO SCIENCE LABORATORIES, Kawasaki-shi, Kanagawa 210-8681 (JP); OSHIMURA, Eiko, AMINO SCIENCE LABORATORIES, Kawasaki-shi, Kanagawa 210-8681 (JP)
(74) Representative: Nicholls, Kathryn Margaret
(86) International application number: JP0102703
(87) International publication number: WO01074305

(57) **Abstract**

A cosmetic composition which comprises one or more kinds of mono-N^{α}-long-chain acylarginines and/or one or more kinds of powders selected from powders subjected to a surface treatment with a mono-N^{α}-long-chain acylarginine and (B) a silicone compound for cosmetics and/or an amphoteric surfactant and is preferably applied to hair or skin. The composition has a feature of imparting gloss and elasticity to hair and skin, whilst little sliminess to skin and scalp during rinsing and little tackiness after drying.

## Description

### Technical Field

The present invention relates to a cosmetic composition. More specifically, the present invention relates to a cosmetic composition that imparts gloss and elasticity to hair and skin, and is with little sliminess to skin and scalp during rinsing and little tackiness after drying.

### Background Art

In a cosmetic composition, particularly in a cosmetic composition for washing hair, an anionic surfactant or an amphoteric surfactant is mainly used as a detergent ingredient. However, these detergent ingredients have a defect of unsuccessful impartment of gloss and elasticity to hair and the like, particularly when the composition is used for fine hair. Hair may sometimes be treated with a conditioning agent composed of a cationic surfactant and an oil to improve conditions of hair. However, since most of the conditioning agent are rinsed away, a problem arises that it is difficult to effectively impart gloss and elasticity to hair after drying. Further, cosmetics for skin are blended with various oils and polyols or various polymers and the like to keep elasticity of skin and clear and smooth complexion. However, their effects are not fully satisfactory.

As for cosmetics for hair, silicone compounds for cosmetics are often blended in shampoos, hair conditioners, or treatment agents to solve these problems, as well as conditioning agents and oils such as stearyltrimethylammonium chloride. However, in shampoos, their effects cannot be successfully obtained, since the silicone compounds are washed away together with surfactants. There is also a problem that, even when the silicone compounds are blended in conditioners or treatment agents, they are washed away together with oils during rinsing, which makes it difficult to effectively impart gloss and elasticity to hair after drying.

As for cosmetics for skin, silicones for cosmetics are blended to improve spreading property at the time of application as well as smoothness and clearness during use. However, addition of silicones for cosmetics alone is not sufficient to improve elasticity and clearness of skin after application.

Japanese Patent Unexamined Publication (Kokai) No. 11-228348 discloses a cosmetic composition containing one or more kinds of mono-N^{α}-long-chain acylarginines and/or powder subjected to a surface treatment with a mono-N^{α}-long-chain acylarginine. This cosmetic composition has excellent conditioning effects such as moistness of hair and hair manageability as well as excellent feeling to skin such as no stiff feeling. However, although moistness to hair and skin, in particular, favorable compatibility of cosmetics for skin (creams), can be imparted by using this powder, gloss and elasticity cannot be imparted. When N^{ε} -lauroyl-L-lysine is added to a hair conditioner or a brushing agent, effects such as improvement of smoothness of hair are obtained. However, no satisfactory effect can be obtained from a viewpoint of impartment of gloss and elasticity to hair.

An amphoteric surfactant used in a detergent composition is useful for improving foaming and detergent properties of an anionic surfactant and obtaining mild feeling of use. However, when an amphoteric surfactant is added to a detergent composition, a problem arises that rinsing property becomes poor during rinsing and sliminess is imparted to skin or scalp, and that tackiness remains after drying. Although various extender pigments may be added to reduce such sliminess during rinsing and tackiness after drying, most portions of the extender pigments are washed away during washing, and it is difficult to effectively reduce sliminess during rinsing.

### Disclosure of the Invention

An object of the present invention is to provide a cosmetic composition. More specifically, the object of the present invention is to provide a cosmetic composition that can impart elasticity and gloss to hair and skin. Another object of the present invention is to provide a detergent composition which is with little sliminess during rinsing and has reduced tackiness after drying when used for washing.

The inventors of the present invention conducted various researches under the circumstances. As a result, they found that the aforementioned objects was achieved by using a mono-N^{α}-long-chain acylarginine and/or a powder composition subjected to a surface treatment with a mono-N^{α}-long-chain acylarginine in combination with a silicone compound for cosmetics and/or an amphoteric surfactant.

The present invention thus provides cosmetic compositions comprising (A) one or more kinds of mono-N^{α}-long-chain acylarginines and/or one or more kinds of powders selected from powders subjected to a surface treatment with a mono-N^{α}-long-chain acylarginine and (B) a silicone compound for cosmetics and/or an amphoteric surfactant.

According to a preferred embodiment of the present invention, provided is the aforementioned cosmetic composition for application to skin or hair.

Among the aforementioned cosmetic compositions, cosmetic compositions containing (A) one or more kinds of mono-N^{α}-long-chain acylarginines and/or one or more kinds of powders selected from powders subjected to a surface treatment with a mono-N^{α}-long-chain acylarginine and (B) a silicone compound for cosmetics has, when applied to hair or skin, a feature of impartation of elasticity and gloss to the hair and skin.

Among the aforementioned cosmetic compositions, compositions containing (A) one or more kinds of mono-N^{α}-long-chain acylarginines and/or one or more kinds of powders selected from powders subjected to a surface treatment with a mono-N^{α}-long-chain acylarginine and (B) an amphoteric surfactant is, when used for washing, almost free from sliminess during rinsing and has reduced tackiness after drying.

From another aspect of the present invention, provided are methods for imparting elasticity and/or gloss to hair or skin, which comprises the step of applying, to hair or skin, a cosmetic composition comprising (A) one or more kinds of mono-N^{α}-long-chain acylarginines and/or one or more kinds of powders selected from powders subjected to a surface treatment with a mono-N^{α}-long-chain acylarginine and (B) a silicone compound for cosmetics; and methods for washing hair or skin, which comprises a step of performing washing by using a composition comprising (A) one or more kinds of mono-N^{α}-long-chain acylarginines and/or one or more kinds of powders selected from powders subjected to a surface treatment with a mono-N^{α}-long-chain acylarginine and (B) an amphoteric surfactant.

From further aspect of the present invention, provided are a use of (A) one or more kinds of mono-N^{α}-long-chain acylarginines and/or one or more kinds of powders selected from powders subjected to a surface treatment with a mono-N^{α}-long-chain acylarginine for manufacture of the aforementioned cosmetic compositions, and a use of (B) a silicone compound for cosmetics and/or an amphoteric surfactant for manufacture of the aforementioned cosmetic compositions,

### Best Mode for Carrying out the Invention

Mono-N^{α}-acylarginines which are usable in the present invention can be obtained by reacting arginine with a long-chain fatty acid halide under an alkaline condition (about pH 12) in a hydrophilic solvent as described in Japanese Patent Unexamined Publication No. 48-23729, or by dehydrating arginine and a salt of fatty acid with heating at a temperature of 100 to 250°C as described in Japanese Patent Unexamined Publication No. 49-1513. Further, as described in Japanese Patent Unexamined Publication No. 11-228527, crystals of mono-N^{α}-acylarginines can be obtained by reacting arginine with a long-chain fatty acid halide in the presence of an alkali (pH 10 to 13) using a mixed solvent of a lower alcohol and/or polyhydric alcohol and water, sufficiently dissolving the product to obtain an acidic or basic aqueous solution, and then adjusting pH of the solution to 5 to 7.

In the present specification, the mono-N^{α}-acylarginines have a straight or branched saturated or unsaturated fatty acid acyl group having 8 to 28 carbon atoms. Examples thereof include mono-N^{α}-oleylarginine, mono-N^{α}-lauroylarginine, mono-N^{α}-myristoylarginine, mono-N^{α}-palmitoylarginine, mono-N^{α}-stearoylarginine, mono-N^{α}-octyldodecylarginine, mono-N^{α}-behenylarginine, mono-N^{α}-(coconut oil fatty acid acyl)arginine, mono-N^{α}-(palm kernel oil fatty acid acyl)arginine, mono-N^{α}-(tallow fatty acid acyl)arginine and the like. Among them, mono-N^{α}-lauroylarginine, mono-N^{α}-myristoylarginine, mono-N^{α}-palmitoylarginine and mono-N^{α}-stearoylarginine are preferred from a viewpoint of feeling of powder. In particular, mono-N^{α}-lauroylarginine, mono-N^{α}-myristoylarginine and mono-N^{α}-palmitoylarginine are more preferred because these compounds are excellent in an effect of imparting moistness to skin. The arginine moiety of mono-N^{α}-acylarginines may be any of D-, L- and DL-arginine. In the cosmetic compositions of the present invention, two or more kinds of mono-N^{α}-acylarginines may be used in combination.

Methods for treatment of surfaces of powder particles for obtaining the powder subjected to a surface treatment with a mono-N^{α}-acylarginine that can be used in the present invention (also referred to as "treated powder" hereinafter in the present specification) are not particularly limited, and may be any of wet treatments or dry treatments, for example. In the wet treatments, the treated powder can be obtained by dissolving powder in a strongly acidic or strongly alkaline solution containing organic solvent, and then neutralizing the solution. In the dry treatment, the treated powder can be obtained by placing powder to be coated and a mono-N^{α}-acylarginine in a pulverizing mixer at several thousands to several tens of thousands revolution per minute to mechanically coat the powder with the mono-N^{α}-acylarginine. A particle size of the treated powder is not particularly limited, and can be appropriately chosen depending on the type of the treated powder.

A type of a material powder for the treated powder usable in the present invention is not particularly limited. Examples thereof include organic powders such as nylon powder, polyethylene powder, polymethyl methacrylate powder, polystyrene powder, styrene/acrylic acid copolymer resin powder, benzoguanamine resin powder, polytetrafluoroethylene powder and cellulose powder, ultraviolet ray blocking powders such as trimethyl-sil-sesquioxane powder, silicone resin powder, talc, kaolin, mica, sericite, muscovite, phlogopite, synthetic mica, lepidolite, lithia mica, vermiculite, magnesium carbonate, calcium carbonate, aluminum silicate, barium silicate, calcium silicate, magnesium silicate, strontium silicate, tungstic acid metal salt, magnesium, silica, zeolite, barium sulfate, calcined calcium sulfate, calcium phosphate, calcium phosphate, apatite hydrofluoride, hydroxyapatite, ceramic powder, zinc myristate, calcium palmitate, aluminum stearate, boron nitride, titanium dioxide, zinc oxide, iron red, iron titanate, fine titanium oxide particles, acicular titanium oxide, spindle-shaped titanium oxide, rod-shaped titanium oxide, fine zinc oxide particles and scaly zinc oxide, pigments such as γ-iron oxide, yellow iron oxide, black iron oxide, carbon black, mango violet, cobalt violet, chromium oxide, cerium oxide, chromium hydroxide, cobalt titanate, ultramarine, iron blue, titanium oxide-coated mica, titanium oxide-coated mica, titanium oxide-coated bismuth oxychloride, bismuth oxychloride, aluminum powder, copper powder and Red No. 201, chlorophyll, β-carotene and the like. Among them, talc, mica, sericite, boron nitride, nylon powder, polyethylene powder, iron red, iron titanate and fine titanium oxide particles are preferred, and talc, mica, sericite and boron nitride are most preferred.

The amount of mono-N^{α}-acylarginine used for the surface treatment of powder is preferably 0.01 to 30% by weight, more preferably 0.1 to 15% by weight, further preferably 0.5 to 5% by weight, based on the weight of the powder to be treated from a viewpoints of economical and uniform surface treatment.

An amount of the mono-N^{α}-acylarginine and/or the powder subjected to a surface treatment with a mono-N^{α}-acylarginine used in the present invention is not particularly limited and can be appropriately determined depending on desired performances of the cosmetic composition. For example, the amount is usually 0.01 to 50% by weight, preferably 0.1 to 10% by weight, more preferably 0.5 to 5% by weight, based on the total weight of the cosmetic composition. Two or more kinds of mono-N^{α}-acylarginines and/or powders subjected to a surface treatment with a mono-N^{α}-acylarginine may be used in combination.

A type of the silicone compound for cosmetics usable in the present invention is not particularly limited. Examples thereof include silicon resin, methylphenylpolysiloxane, methylpolysiloxane, octamethylcyclotetrasiloxane, dimethylsiloxane/methyl(polyoxyethylene)siloxane copolymer, decamethylcyclopentanesiloxane, dimethylsiloxanemethyl(polyoxyethylene/polyoxypropylene)siloxane copolymer, methyl hydrogen polysiloxane, dodecamethylcyclohexasiloxane, methylpolycyclosiloxane, dimethylsiloxane/methylstearoxysiloxane copolymer, methylpolysiloxane emulsion, octamethyltrisiloxane, cyclic silicon resin, highly polymerized methylpolysiloxane, tetradecamethylhexasiloxane, trimethylsiloxysilicate and so forth. Among these, methylphenylpolysiloxane, methylpolysiloxane, octamethylcyclotetrasiloxane, dimethylsiloxane/methyl(polyoxyethylene)siloxane copolymer, dimethylsiloxanemethyl(polyoxyethylene/polyoxypropylene)siloxane copolymer and dodecamethylcyclohexasiloxane are preferred, and dimethylsiloxane/methyl(polyoxyethylene)siloxane copolymer is most preferred.

An amount of silicone for cosmetics used in the present invention is not particularly limited and can be appropriately determined depending on desired performances of the cosmetic composition. For example, the amount is usually 0.01 to 30% by weight, preferably 0.1 to 10% by weight, more preferably 0.2 to 5% by weight, based on the total weight of the cosmetic composition. Two or more kinds of silicones for cosmetics may be used in combination.

A type of the amphoteric surfactant used in the present invention is not particularly limited. Examples thereof include amphoteric surfactants such as carbobetaine-type amphoteric surfactants, amidobetaine-type amphoteric surfactants, sulfobetaine-type amphoteric surfactants, hydroxysulfobetaine-type amphoteric surfactants, amidosulfobetaine-type amphoteric surfactants, phosphobetaine-type amphoteric surfactants and imidazoline-type amphoteric surfactants. More specific examples include lauryldimethylaminoacetic acid betaine, (coconut oil alkyl)betaine, stearyldimethylaminoacetic acid betaine, stearyldihydroxyethylbetaine, (lauric acid amido)propylbetaine, (coconut oil fatty acid amido)propylbetaine solution, (palm kernel oil fatty acid amido)propylbetaine solution, (ricinoleic acid amido)propylbetaine solution, N-(coconut oil fatty acid acyl)-N-carboxyethyl-N-hydroxyethylethylenediamine salt, (palm kernel oil fatty acid acyl)-N-carboxyethyl-N-hydroxyethylethylenediamine salt, 2-alkyl-N-carboxymethyl-N-hydroxyethylimidazolium betaine, undecyl-N-carboxymethylimidazolinium betaine, alkyldiaminoethylglycine hydrochloride solution, bis(stearyl-N-hydroxyethylimidazoline)/chloracetic acid complex and the like.

An amount of the amphoteric surfactant used in the present invention is not particularly limited and can be appropriately determined depending on desired performances of the cosmetic composition. For example, the amount is usually 0.01 to 30% by weight, preferably 0.1 to 15% by weight, more preferably 0.2 to 10% by weight, based on the total weight of the cosmetic composition. Two or more kinds of amphoteric surfactants may be used in combination.

A type of intended use of the cosmetic composition of the present invention is not particularly limited. Preferably, the composition can be used as a cosmetic composition for skin or hair. For example, the composition can be used as various cosmetics for hair such as shampoo, hair conditioner, hair conditioner-in shampoo, conditioning shampoo, hair lotion, hair conditioner, hair treatment agent, hair cream, hair spray, hair liquid, hair wax, hair water, hair styling agent, permanent wave agent, hair coloring agent, acidic hair coloring agent and hair manicure and various cosmetics for skin such as skin lotion, emulsified lotion, face wash, make-up remover, cleansing lotion, emollient lotion, nourishing cream, emollient cream, massage cream, cleansing cream, body-shampoo, hand-wash liquid soap, solid soap, shaving cream, cosmetics for suntan, deodorant powder, deodorant lotion, deodorant spray, make-up removing gel, moisture gel, moisturizing essence, UV-cut essence, shaving foam, white powder, foundation, lip color, cheek color, eye liner, eye shadow, eyebrow cosmetics and bath liquid. Further, the cosmetic composition of the present invention can also be used as toothpaste or the like.

The cosmetic composition of the present invention can be added with various kinds of ordinarily used additives so long as the effects of the present invention are not degraded. Such additives are widely used in the field of cosmetics and can be appropriately selected by those skilled in the art. Examples thereof include materials described in the Japanese Standards of Cosmetic Ingredients, Comprehensive Licensing Standards of Cosmetics by Category, the Japanese Standards of Quasi-Drugs, the Japanese Pharmacopoeia, the Japan's Specifications and Standards for Food Additives and the like, such as surfactants including anionic surfactants, cationic surfactants and nonionic surfactants, waxes, vegetable oils, animal fats and oils, natural fat and oil derivatives, mineral fats and oils, lower and higher fatty acid esters, synthetic fats and oils such as N-acyl glutamic acid ester, polymer substances, alcohols, polyhydric alcohols, extracts, amino acids, nucleic acids, vitamins, hydrolyzed proteins and derivatives thereof, glyceryl oleate, enzymes, anti-inflammatory agents, antibacterial agents, antiseptics, antioxidants, ultraviolet absorbers, chelating agents, antiperspirants, oxidation dyes, pH modifiers, pearling agents and moistening agents. Two or more kinds of these additives may be used in combination.

The entire disclosures of the specification and claims of Japanese Patent Application No. 2000-100322 are herein incorporated by reference.

### Examples

The present invention will be explained more specifically with reference to the following examples. However, the scope of the present invention is not limited to these examples.

### <Preparation Example 1>

### Synthesis of N^{α}-lauroyl-L-arginine

L-Arginine (1106 g) was added with isopropyl alcohol (6919 g) and water (2964 g), and added dropwise simultaneously with lauryl chloride (1522 g, NOF Corporation) and 27 weight % aqueous NaOH with stirring over 2 hours while pH was maintained at 10.5 to 11.5 and a reaction temperature at 10 to 13°C. The reaction mixture was ripened for 1 hour, then heated to 50°C and added with concentrated hydrochloric acid to adjust pH to 3.8 to completely dissolve the reaction product. Then, the reaction mixture was added with 27 weight % aqueous NaOH to adjust pH to 6.0 to precipitate crystals, and the resulting slurry was gradually cooled to 10°C with stirring. The slurry was cooled to 10°C and filtered. The crystals were washed with water (10 kg) and isopropyl alcohol (4.4 kg). The resulting crystals were dried under reduced pressure to obtain scaly crystals of mono-N^{α}-lauryl-L-arginine (2081 g, yield: 92.3%). The decomposition temperature of the mono-N^{α}-lauryl-L-arginine obtained was about 164°C.

### <Preparation Example 2>

### Surface treatment of talc with N^{α}-lauroyl-L-arginine

Talc (5.0 g) was added with the compound (0.05 g) of Preparation Example 1 and mixed twice for 1 minute with stirring by using a mixer for household use for surface treatment to obtain a treated powder.

### <Test Example 1>

Shampoos each having the composition shown in Table 1 (amounts of ingredients are shown in weight % relative to the total weight of 100%) were prepared in a conventional manner, and applied to hair bundles washed with 1% aqueous sodium lauryl ether sulfate, and then the hair bundles were sufficiently rinsed with water. Sensory evaluation was performed for (a) gloss of hair and (b) elasticity of hair after drying. The evaluation was performed as relative evaluation on the basis of Comparative Example 1 shown in Table 1 as a reference standard, and average values of scores according to the criteria shown below were calculated. An average value of 4.5 or higher was represented as very good (ⓞ), 3.5 to 4.4 as good (○), 2.5 to 3.4 as normal (Δ), and 2.4 or less as poor (×). The evaluation results are shown in Table 1.

### <Criteria>

(a) Gloss after drying
5: Glossier than reference standard
4: Slightly glossier than reference standard
3: Same level as reference standard
2: Slightly less glossy than reference standard
1: Less glossy than reference standard

(b) Elasticity after drying
5: More elastic than reference standard
4: Slightly more elastic than reference standard
3: Same level as reference standard
2: Slightly less elastic than reference standard
1: Less elastic than reference standard

**Table 1**

| | Comparative Examples | | | | | Examples | | | | | |
|---|---|---|---|---|---|---|---|---|---|---|---|
| | 1 | 2 | 3 | 4 | 5 | 1 | 2 | 3 | 4 | 5 | 6 |
| Lauryl ether sulfate ammonium salt (2EO, 25%) | 50 | 50 | 50 | 50 | 50 | 50 | 50 | 50 | 50 | 50 | 50 |
| Mono-N^{α}-lauroylarginine | | | | 1 | | 1 | 1 | 1 | | | |
| Compound of Production Example 2 | | | | | | | | | 1 | 1 | 1 |
| Dimethylsiloxane/methyl (polyoxyethylene) siloxane copolymer (*1) | 1 | | | | 1 | 1 | | | 1 | | |
| Dodecamethylcyclohexasiloxane | | 1 | | | | | 1 | | | 1 | |
| Methylpolysiloxane (10,000 cPs) | | | 1 | | | | | 1 | | | 1 |
| Talc | | | 1 | | 1 | | | | | | |
| N^{ε} -lauroyl-L-lysine (*2) | 1 | | | | | | | | | | |
| Purified water | r.q. | r.q. | r.q. | r.q. | r.q. | r.q. | r.q. | r.q. | r.q. | r.q. | r.q. |
| Gloss of hair after drying | Δ | ○ | × | × | × | ○ | ○ | ○ | ○ | ○ | ○ |
| Elasticity of hair after drying | Δ | × | × | ○ | × | ○ | ○ | ○ | ○ | Δ | Δ |

| | | | | | | | | | | | |
|---|---|---|---|---|---|---|---|---|---|---|---|
| (*1) TSF4440 produced by Toshiba Silicone Co., Ltd. | | | | | | | | | | | |
| (*2) AMIHOPE LL produced by Ajinomoto Co., Ltd. r.q.: residual quantity | | | | | | | | | | | |

### <Test Example 2>

Conditioners each having the composition shown in Table 2 (amounts of ingredients are shown in weight % based on the total amount of 100%) were prepared and applied to hair bundles from Chinese subjects washed with 1% aqueous sodium lauryl ether sulfate, and the hair bundles were sufficiently rinsed with water. Sensory evaluation was performed about (a) elasticity of hair and (b) gloss of hair after drying. The evaluation was performed as relative evaluation on the basis of Comparative Example 6 shown in Table 2 as a reference standard, and using the same criteria as in Test Example 1. The evaluation results are shown in Table 2.

**Table 2**

| | Comparative Examples | | | | Examples | | | | | |
|---|---|---|---|---|---|---|---|---|---|---|
| | 6 | 7 | 8 | 9 | 7 | 8 | 9 | 10 | 11 | 12 |
| Stearyltrimethylammonium chloride (*1) | 1 | 1 | 1 | 1 | 1 | 1 | 1 | 1 | 1 | 1 |
| Cetanol | 3 | 3 | 3 | 3 | 3 | 3 | 3 | 3 | 3 | 3 |
| Octyldodecyl myristate | 2 | 2 | 2 | 2 | 2 | 2 | 2 | 2 | 2 | 2 |
| Methylparaben | 0.2 | 0.2 | 0.2 | 0.2 | 0.2 | 0.2 | 0.2 | 0.2 | 0.2 | 0.2 |
| Mono-N^{α}-cocoylarginine | | | | 1 | 1 | 1 | 1 | | | |
| Compound of Production Example 2 | | | | | | | | 1 | 1 | 1 |
| Dimethylsiloxane/methyl (polyoxyethylene)siloxane copolymer (*2) | | | 1 | | 1 | | | 1 | | |
| Dodecamethylcyclohexa siloxane | | 1 | | | | 1 | | | 1 | |
| Methylpolysiloxane (10,000 cPs) | 1 | | | | | | 1 | | | 1 |
| Talc | | 1 | 1 | | | | | | | |
| N^{ε}-lauroyl-L-lysine (*3) | 1 | | | | | | | | | |
| Purified water | r.q. | r.q. | r.q. | r.q. | r.q. | r.q. | r.q. | r.q. | r.q. | r.q. |
| Gloss of hair after drying | Δ | × | × | × | ○ | ○ | ○ | ○ | ○ | ○ |
| Elasticity of hair after drying | Δ | × | × | Δ | ○ | ○ | ○ | ○ | Δ | Δ |

| | | | | | | | | | | |
|---|---|---|---|---|---|---|---|---|---|---|
| (*1) QUARTAMIN 86P CONC. produced by Kao Corporation | | | | | | | | | | |
| (*2) TSF4440 produced by Toshiba Silicone Co., Ltd. | | | | | | | | | | |
| (*3) AMIHOPE LL produced by Ajinomoto Co., Ltd. r.q.: residual quantity | | | | | | | | | | |

### <Test Example 3>

Creams each having the composition shown in Table 3 (amounts of ingredients are shown in weight % based on the total weight of 100%) were prepared and applied to back of hands of a panel of 5 experts. Sensory evaluation was performed about (a) clear and smooth complexion after application and compatible of the cream to the skin and (b) elasticity of skin after use. The evaluation was performed as relative evaluation on the basis of Comparative Example 10 in Table 3 as a reference standard, and average values of scores according to the criteria shown below were calculated. An average value of 4.5 or higher was represented as very good (ⓞ), 3.5 to 4.4 as good (○), 2.5 to 3.4 as normal (Δ), and 2.4 or less as poor (×). The evaluation results are shown in Table 3.

### <Criteria>

(a) Clearness at application after the application and compatibleness to skin
   5: Clearer than reference standard
   4: Slightly clearer than reference standard
   3: Same level as reference standard
   2: Slightly less clear than reference standard
   1: Less clear than reference standard
(b) Elasticity of skin after use
   5: More elastic than reference standard
   4: Slightly more elastic than reference standard
   3: Same level as reference standard
   2: Slightly less elastic than reference standard
   1: Less elastic than reference standard

**Table 3**

| | Comparative examples | | | | Examples | |
|---|---|---|---|---|---|---|
| | 10 | 11 | 12 | 13 | 13 | 14 |
| Liquid paraffin | 36 | 36 | 36 | 36 | 36 | 36 |
| Cetanol | 4.5 | 4.5 | 4.5 | 4.5 | 4.5 | 4.5 |
| Sorbitan monostearate | 1.8 | 1.8 | 1.8 | 1.8 | 1.8 | 1.8 |
| Polyoxyethylene (20) Sorbitan monoolate | 2.8 | 2.8 | 2.8 | 2.8 | 2.8 | 2.8 |
| Mono-N^{α}-stearoylarginine | | | | 1 | 1 | |
| Compound of Production Example 2 | | | | | | 1 |
| Methylpolysiloxane (1,000 cPs) | 1 | 1 | | | 1 | 1 |
| N^{ε}-lauroyl-L-lysine (*1) | 1 | | 1 | | | |
| Purified water | r.q. | r.q. | r.q. | r.q. | r.q. | r.q. |
| Gloss of skin after drying | Δ | Δ | × | Δ | ○ | ○ |
| Elasticity of skin after drying | Δ | × | Δ | Δ | ○ | Δ |

| | | | | | | |
|---|---|---|---|---|---|---|
| (*1) AMIHOPE LL produced by Ajinomoto Co., Ltd. r.q.: residual quantity | | | | | | |

### <Preparation of shampoo>

A shampoo was prepared according to the following composition in a conventional manner.

| | |
|---|---|
| Polyoxyethylene lauryl ether sulfate (25%) | |
| (EMAL 20C: Kao) | 10.0% |
| Alkylpolyglycoside (50%) | |
| (PLANTACARE 2000UP: Cognis) | 8.0% |
| Cocoamidopropylbetaine (30%) | 10.0% |
| Polyoxyethylene (2) lauryl ether | 1.0% |
| Polyether-denatured silicone | |
| (TSF44452: Toshiba Silicone) | 1.5% |
| Mono-N^{α}-cocoylarginine | 0.7% |
| Purified water | residual quantity |

The shampoo prepared was excellent in impartation of moistness after washing as well as impartation of gloss and elasticity to hair, which are the effects of the present invention.

### <Preparation of hair treatment agent>

A hair treatment agent was prepared according to the following composition in a conventional manner.

| | |
|---|---|
| Cetostearyl alcohol | 7.0% |
| Isocetyl myristate | 2.0% |
| N-[Alkyl(12,14)oxy-2-hydroxypropyl]-L-arginine hydrochloride solution | (30%) |
| | 2.0% |
| Di(phytosteryl 2-octyldodecyl) N-lauroyl-L-glutamate | |
| | 0.01% |
| "PCA Soda" (Ajinomoto) | 2.0% |
| Lactic acid (90%) | 0.05% |
| Methyl paraoxybenzoate | 0.2% |
| Citric acid | as required |
| Purified water | residual quantity |
| Highly polymerized methylpolysiloxane | 1.5% |
| Trimethylsilylamodimethicon | |
| (XS-65-A2593: Toshiba Silicone) | 2.0% |
| N-[2-Hydroxy-3-(trimethylammonio)propyl] chloride | |
| hydrolyzed collagen | 1.0% |
| Mono-N^{α}-lauroylarginine | 0.3% |
| Perfume | as required |

The treatment agent prepared had a smooth texture and was excellent in impartation of gloss and elasticity after rinsing.

### <Preparation of hair conditioner>

A hair conditioner was prepared according to the following composition in a conventional manner.

| | |
|---|---|
| Cetostearyl alcohol | 4.0% |
| Isocetyl isostearate | 2.0% |
| POE (20) behenyl ether | 2.0% |
| Phenoxy ethanol | 0.6% |
| Cocoylarginine ethyl ester pyrrolidonecarboxylic | |
| acid salt ("CAE", Ajinomoto) | 0.5% |
| Lactic acid (90%) | 0.05% |
| Purified water | residual quantity |
| 30% hydrolyzed collagen | 2.0% |
| Trimethylsiloxysilicate | 1.0% |
| Trimethyl glycine | 3.0% |
| Purified water | residual quantity |
| Mono-N^{α}-stearoylarginine | 1.0% |
| Perfume | as required |

The conditioner prepared extended favorably when applied to hair, and was also excellent in impartation of gloss and elasticity to hair after applied to hair and dried.

### <Test Example 4>

Shampoos each having the composition shown in Table 4 (amounts of ingredients are shown in weight % based on the total weight of 100%) were prepared in a conventional manner and applied to hair bundles washed with 1% aqueous sodium lauryl ether sulfate, and the hair bundles were sufficiently rinsed with water. Sensory evaluation was performed about (a) time required for rinsing and (b) tackiness after drying. The results of evaluation are shown in Table 4. The evaluation was performed according to the criteria shown bellow.

### <Evaluation criteria>

(a) Time required for rinsing
   ○: Favorable
   Δ: Slightly long time required for rinsing
   ×: Long time required for rinsing with sliminess
(b) Tackiness after drying
   ○: No tackiness
   Δ: Slight tackiness
   ×: Much tackiness

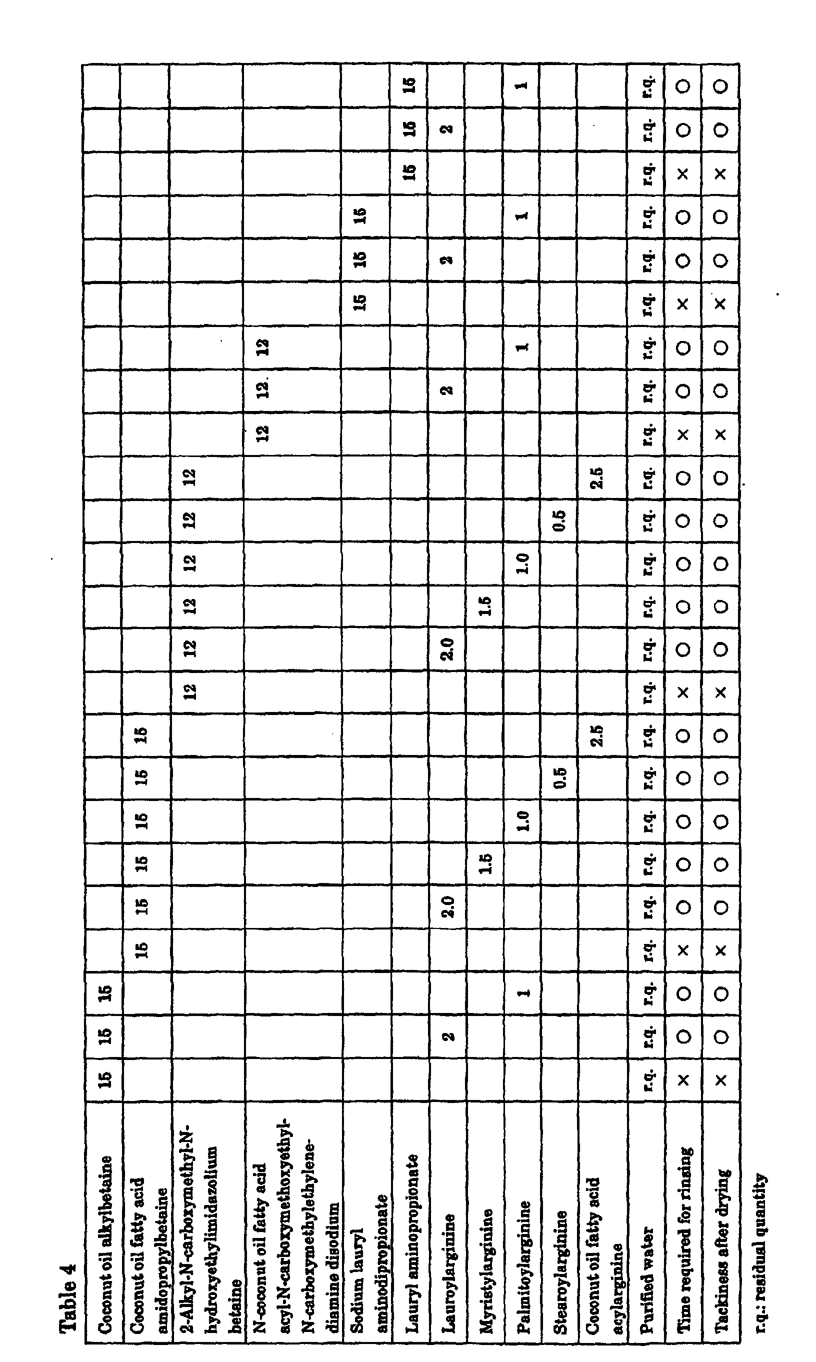

### Industrial Applicability

According to the present invention, a cosmetic composition is provided which can impart gloss and elasticity to hair and skin, whilst little sliminess to skin or scalp during rinsing and little tackiness after drying.

## Claims

1. A cosmetic composition which comprises (A) one or more kinds of mono-N^{α}-long-chain acylarginines and/or one or more kinds of powders selected from powders subjected to a surface treatment with a mono-N^{α}-long-chain acylarginine and (B) a silicone compound for cosmetics and/or an amphoteric surfactant.

2. The cosmetic composition according to Claim 1, which is applied to skin or hair.
